Europäisches Patentamt

European Patent Office

Office européen des brevets

(19)

(11) **EP 0 525 843 B1**

(12) **FASCICULE DE BREVET EUROPEEN**

(45) Date de publication et mention
de la délivrance du brevet:
**23.04.1997 Bulletin 1997/17**

(51) Int. Cl.6: **B01J 31/02**, B01J 31/30,
C07C 17/08

(21) Numéro de dépôt: **92201677.9**

(22) Date de dépôt: **10.06.1992**

(54) **Système catalytique d'hydrochloration et procédé de fabrication de chlorure de vinyle au départ d'acétylène et de chlorure d'hydrogène en présence de ce système catalytique**

Hydrochlorierung von katalytischen Systemen und Verfahren zur Herstellung von Vinylchlorid aus Acetylen und Chlorwasserstoff in Gegenwart dieses katalytischen Systems

Hydrochlorination catalyst system and preparation process of vinyl chlorided from acetylene and hydrogen chloride in presence of this catalyst system

(84) Etats contractants désignés:
**AT BE DE ES FR GB GR IT NL PT SE**

(30) Priorité: **20.06.1991 BE 9100600**

(43) Date de publication de la demande:
**03.02.1993 Bulletin 1993/05**

(73) Titulaire: **SOLVAY (Société Anonyme)
B-1050 Bruxelles (BE)**

(72) Inventeurs:
- **Strebelle, Michel
B-1150 Bruxelles (BE)**
- **Devos, André
B-1940 Sint-Stevens-Woluwe (BE)**

(74) Mandataire: **Jacques, Philippe et al
Solvay S.A.
Département Propriété Industrielle
310, rue de Ransbeek
1120 Bruxelles (BE)**

(56) Documents cités:
**EP-A- 0 090 443          EP-A- 0 340 416
DE-A- 3 500 318          DE-C-  709 000
GB-A- 2 001 546**

- **CHEMICAL ABSTRACTS, vol. 71, no. 3, 1969,
Columbus, Ohio, US; abstract no. 12510v, N.
KARAPETYAN 'CATALYST FOR SYNTHESIS OF
VINYL CHLORIDE FROM ACETYLENE' page 269**
**;**

EP 0 525 843 B1

## Description

La présente invention concerne un système catalytique liquide d'hydrochloration à base d'un composé de métal du groupe VIII et un procédé de fabrication de chlorure de vinyle par hydrochloration de l'acétylène en présence d'un tel système catalytique.

La fabrication du chlorure de vinyle par réaction entre l'acétylène et le chlorure d'hydrogène est classiquement réalisée en phase gazeuse, dans un réacteur à lit fixe, en présence d'un catalyseur solide hétérogène à base de chlorure de mercure sur support. Principalement pour des raisons de toxicité, il existe actuellement un intérêt croissant pour des systèmes catalytiques exempts de composés du mercure. Divers catalyseurs destinés à remplacer les catalyseurs actuels dans les procédés en phase gazeuse ont été développés. Par exemple, la demande de brevet japonais non examinée 52/136104 décrit un procédé d'hydrochloration de l'acétylène en phase gazeuse en présence d'un lit fixe de catalyseur constitué d'halogénures de métaux nobles déposés sur charbon actif. Jusqu'à présent cependant, la durée de vie de tels catalyseurs alternatifs destinés à des procédés en phase gazeuse reste très inférieure à celle des catalyseurs à base de composés du mercure.

Il existe par ailleurs dans la littérature quelques exemples d'hydrochloration de l'acétylène en présence d'un milieu catalytique liquide. Le brevet allemand 709.000 décrit un procédé de préparation d'halogénures de vinyle par mise en contact à température élevée d'acétylène avec une masse fondue de sels d'halogénohydrates de bases organiques renfermant un catalyseur usuel. Comme bases organiques sont envisagées les amines aliphatiques, aromatiques ou hétérocycliques et leurs mélanges. Dans l'exemple 1, du chlorure de vinyle est obtenu par dispersion de chlorure d'hydrogène et d'acétylène dans un mélange constitué de 350 parties en volume de pyridine, de 350 parties en volume de diéthylamine et de 100 parties en poids de chlorure de mercure, maintenu à 220-225° C. Le certificat d'auteur SU-237116 décrit l'emploi d'une solution aqueuse acide contenant 46 % en poids de chlorure cuivreux et de 14 à 16 % en poids d'un chlorhydrate de méthyl-, diméthyl- ou triméthylamine. La demande de brevet japonais non examinée 52/136103 divulgue un procédé de préparation du chlorure de vinyle par réaction d'acétylène avec du chlorure d'hydrogène en présence d'un catalyseur préparé par mise en suspension dans l'eau ou dans un solvant organique d'un système binaire sélectionné parmi le chlorure d'or, le chlorure de platine et le chlorure de palladium, éventuellement modifié par un chlorure d'un métal de transition à valence variable. La demande de brevet EP-A-0340416 divulgue un procédé de préparation du chlorure de vinyle par réaction d'acétylène avec du chlorure d'hydrogène en présence d'un composé de palladium comme catalyseur dans un solvant constitué par un amide aliphatique ou cycloaliphatique, à une température supérieure à la température ambiante. Bien qu'il permette d'atteindre des rendements élevés, il est apparu que, dans les conditions de réaction, ce système catalytique se dégrade progressivement, en formant des produits noirâtres d'apparence charbonneuse.

En conséquence, l'invention a pour but un système catalytique d'hydrochloration stable, exempt de composés du mercure. L'invention a encore pour but de fournir un procédé de synthèse du chlorure de vinyle par hydrochloration de l'acétylène en présence d'un tel système catalytique liquide qui est particulièrement actif, et/ou qui ne se dégrade pas dans les conditions réactionnelles et qui permet en outre d'obtenir le chlorure de vinyle avec une grande sélectivité et donc de diminuer fortement la quantité de sous-produits à éliminer. A la différence des systèmes à base de composés du mercure, le système catalytique selon l'invention présente de plus l'avantage d'éviter la vaporisation de sels métalliques dans l'installation.

L'invention concerne un système catalytique liquide d'hydrochloration, plus particulièrement d'hydrochloration de l'acétylène. Ce système catalytique comprend au moins un composé de métal du groupe VIII, un chlorhydrate d'amine grasse dont le point de fusion est supérieur à 25° C et un solvant organique. Par amine grasse, on entend toute amine ou mélanges d'amines contenant un nombre élevé d'atomes de carbone, par exemple, plus de 8 atomes de carbone, présentant une structure moléculaire peu ou pas ramifiée. Les amines préférées sont celles qui renferment de 10 à 20 atomes de carbone. Cette structure moléculaire peu ou pas ramifiée permet une cristallisation aisée du chlorhydrate formé par réaction de l'amine grasse avec le chlorure d'hydrogène et explique les points de fusion élevés des chlorhydrates de ces composés. Des amines répondant à la définition d'amine grasse ci-dessus sont, par exemple, la décylamine, l'undécylamine, la dodécylamine, la 3-méthyldodécylamine.

De bons résultats ont été obtenus avec un système catalytique comprenant le chlorhydrate de dodécylamine.

Les composés de métaux du groupe VIII mis en oeuvre dans les systèmes catalytiques de la présente invention sont généralement choisis parmi les composés de fer, de cobalt, de nickel, de ruthénium, de rhodium, de palladium, d'osmium, d'iridium, de platine ou de leurs mélanges. Les chlorures de ces métaux du groupe VIII sont préférés, mais tout autre composé pouvant se transformer en chlorure en présence de chlorure d'hydrogène lors de la préparation du système catalytique peut aussi être utilisé. De préférence, le composé de métal du groupe VIII mis en oeuvre dans la présente invention est choisi parmi les composés de platine et les composés de palladium, tels que le chlorure de platine(II) ou le chlorure de palladium(II), un platinochlorure ou un palladochlorure des métaux alcalins ou des métaux alcalinoterreux - par exemple, $Na_2(PtCl_4)$, $Na_2(PdCl_4)$, $K_2(PtCl_4)$, $K_2(PdCl_4)$, $Li_2(PtCl_4)$, $Li_2(PdCl_4)$, $(NH4)_2(PtCl_4)$ et $(NH4)_2(PdCl_4)$ -, l'acide hexachloroplatinique ou ses sels, par exemple $Na_2PtCl_6$, $K_2PtCl_6$, $Li_2PtCl_6$, les composés de palladium dans lesquels le palladium a une valence élevée, comme $Na_2PdCl_6$, $K_2PdCl_6$, $Li_2PdCl_6$, et... On peut aussi

EP 0 525 843 B1

mettre en oeuvre des complexes des métaux du groupe VIII dans lesquel le métal a la valence O, tels que les complexes Pt(P$\phi_3$)$_2$, Pd(P$\phi_3$)$_2$, (P$\phi_3$)Pt(CO), etc... Des mélanges de composés de métaux du groupe VIII peuvent aussi être utilisés.

Les composés de métaux du groupe VIII particulièrement préférés sont le chlorure de platine(II) et le chlorure de palladium(II). Le composé de métal du groupe VIII le plus particulièrement préféré est le chlorure de palladium(II).

Le choix de la nature du solvant organique mis en oeuvre dans le procédé selon l'invention est conditionné notamment par la nécessité qu'il soit inerte vis-à-vis des réactifs dans les conditions de réaction, qu'il soit miscible avec le chlorhydrate d'amine grasse à la température de réaction et qu'il soit capable de solubiliser celui-ci à une température inférieure à son point de fusion. Par ailleurs, pour des raisons de sécurité et de facilité d'emploi, on donne la préférence aux solvants organiques peu volatils. Le choix du solvant organique est aussi influencé par sa capacité d'absorption de l'acétylène. Les solvants satisfaisant aux différents critères exposés ci-dessus sont choisis parmi les hydrocarbures aliphatiques, cycloaliphatiques et aromatiques et leurs mélanges, par exemple les paraffines en C7 à C15 et les alkylbenzènes, notamment les xylènes, les propylbenzènes, les butylbenzènes, les méthyléthylbenzènes. Pour des considérations de nature économique, le solvant mis en oeuvre est de préférence choisi parmi les produits commerciaux constitués de mélanges d'hydrocarbures aliphatiques tels que le solvant ISOPAR de Esso ou le solvant SHELLSOL K de Shell ou de mélanges de composés aromatiques tels que le solvant SOLVESSO de Esso ou le solvant SHELLSOL AB de Shell.

Des solvants ayant donné de bons résultats sont les solvants aliphatiques saturés, tels que le solvant SHELLSOL K, constitué de coupes pétrolières ayant un point d'ébullition compris entre environ 190° C et environ 250° C.

D'autres solvants envisageables sur base des divers critères donnés ci-dessus sont certains composés halogénés lourds, tels que des halogénoalcanes, halogénobenzènes et autres dérivés halogénés de composés aromatiques.

Le système catalytique le plus particulièrement préféré contient le chlorhydrate de dodécylamine, du chlorure de palladium(II) et un solvant aliphatique tel que le solvant SHELLSOL K. Un tel système catalytique possède une grande activité catalytique et une sélectivité en chlorure de vinyle qui peut dépasser 99 %. De plus, ce système ne se dégrade quasi pas au cours du temps.

Le rapport pondéral entre le solvant organique et le chlorhydrate d'amine grasse est généralement supérieur à environ 0,1. De préférence, ce rapport est supérieur ou égal à environ 0,5. Dans les conditions particulièrement préférées, il est supérieur ou égal à environ 0,8. Généralement, ce rapport est inférieur ou égal à environ 20. De préférence, il est inférieur ou égal à environ 10. Dans les conditions particulièrement préférées, il est inférieur ou égal à environ 8.

La teneur en composé de métal du groupe VIII dans le système catalytique, exprimée en millimoles par litre de solution de système catalytique est généralement supérieure ou égale à environ 1 mmol/l, de préférence supérieure ou égale à environ 10 mmol/l. La teneur en composé de métal du groupe VIII dans le système catalytique est généralement inférieure ou égale à environ 200 mmol/l, de préférence inférieure ou égale à environ 100 mmol/l. Bien que cela ne soit pas indispensable, il est cependant préférable que tout le composé de métal du groupe VIII compris dans le système catalytique soit sous forme dissoute. Généralement, le système catalytique est préparé par dissolution ou dispersion de la quantité voulue de composé de métal du groupe VIII dans l'amine grasse ou dans le mélange amine grasse / solvant organique, par chauffage de cette solution à une température supérieure à la température de fusion du chlorhydrate d'amine grasse, puis par saturation de cette solution par du chlorure d'hydrogène provoquant la formation du chlorhydrate d'amine grasse. Il est cependant également possible, bien que moins aisé pratiquement, de d'abord saturer l'amine grasse ou le mélange amine grasse / solvant organique, préalablement chauffé, avec du chlorure d'hydrogène afin de former le chlorhydrate d'amine grasse, puis d'introduire ensuite le composé de métal du groupe VIII dans le chlorhydrate d'amine grasse ou dans le mélange de celui-ci avec le solvant organique. Habituellement, la quantité de composé de métal du groupe VIII mise en oeuvre est telle que, dans le système catalytique, tout le composé de métal du groupe VIII se trouve sous formé dissoute. On peut cependant aussi mettre en oeuvre un composé de métal du groupe VIII en quantité ou de nature telle qu'une fraction au moins de ce composé soit présente dans le système catalytique sous forme solide dispersé, sans porter préjudice à l'invention.

L'invention concerne également un procédé de fabrication de chlorure de vinyle par hydrochloration de l'acétylène en présence d'un système catalytique liquide comprenant au moins un composé de métal du groupe VIII, un chlorhydrate d'amine grasse dont le point de fusion est supérieur à 25° C et un solvant organique. La nature et les proportions des constituants du système catalytique mis en oeuvre dans le procédé selon l'invention sont celles définies ci-dessus.

Le procédé selon l'invention est réalisable de la température ambiante jusqu'à environ 200° C. A plus haute température, le système catalytique a tendance à se dégrader rapidement. Généralement, la température de réaction est telle que tout le chlorhydrate d'amine grasse est en solution. La température de réaction préférée, c'est-à-dire celle offrant le meilleur compromis entre productivité, rendement et stabilité du milieu catalytique est supérieure ou égale à environ 80° C. Les meilleurs résultats sont obtenus à des températures supérieures ou égales à environ 120° C. De préférence, la température de réaction ne dépasse pas environ 180° C. Une température de réaction inférieure ou égale à environ 170° C est particulièrement préférée. Le procédé selon l'invention est généralement effectué à la pression atmosphérique ou à une pression légèrement supérieure compatible avec les règles de sécurité de manipulation de l'acétylène, c'est-à-dire ne dépassant pas environ 1,5 bar.

3

Le procédé de fabrication de chlorure de vinyle par hydrochloration de l'acétylène selon l'invention est réalisé par mise en contact dans tout réacteur approprié, des réactifs gazeux - acétylène et chlorure d'hydrogène - avec le système catalytique liquide. Le procédé selon l'invention peut être réalisé classiquement dans tout appareillage favorisant l'échange gaz-liquide, tel qu'une colonne à plateaux ou une colonne noyée à empilages. Un autre mode de mise en oeuvre du procédé permettant de bons échanges de matière entre les phases liquide et gazeuse consiste en l'emploi d'un réacteur à contre-courant, éventuellement du type empilages à lit arrosé, le système catalytique liquide ruisselant sur les empilages, à contre-courant du flux gazeux des réactifs.

Dans le procédé selon l'invention, le rapport molaire entre le chlorure d'hydrogène et l'acétylène introduits dans le réacteur est en général supérieur ou égal à environ 0,5. De préférence, ce rapport est supérieur ou égal à environ 0,8. En général, ce rapport molaire est inférieur ou égal à environ 3. De bons résultats ont été obtenus avec un rapport molaire entre le chlorure d'hydrogène et l'acétylène introduits dans le réacteur inférieur ou égal à environ 1,5. L'acétylène et le chlorure d'hydrogène peuvent être mis en contact dans le réacteur ou, de préférence, mélangés préalablement à leur introduction dans le réacteur.

Dans le but d'augmenter la quantité d'acétylène dissous dans la phase catalytique liquide, il est également possible de mettre en oeuvre un procédé dans lequel seul l'acétylène est introduit dans le réacteur sous forme gazeuse, où il réagit avec le chlorure d'hydrogène présent dans la phase liquide sous forme de chlorhydrate, le chlorhydrate d'amine grasse du système catalytique étant régénéré par mise en contact d'une navette liquide renfermant l'amine grasse avec du chlorure d'hydrogène en dehors du réacteur.

L'invention est illustrée par les exemples suivants. Les exemples 1 à 5 sont réalisés selon l'invention. Les exemples 6(C) à 8(C) sont réalisés à titre de comparaison.

Exemples 1 à 3

Le système catalytique est préparé au départ de dodécylamine, de chlorure de palladium(II) et de solvant SHELLSOL K.

Le solvant SHELLSOL K est un produit commercialisé par Shell, constitué d'un mélange d'hydrocarbures, essentiellement de nature aliphatique. Le produit utilisé dans ces exemples a un point d'ébullition initial de 193° C et un point d'ébullition final de 245° C.

La dodécylamine est d'abord mélangée à des quantités variables de solvant SHELLSOL K, puis 4 g de chlorure de palladium(II), soit 22,6 mmol, sont introduits sous agitation dans un litre de solution. Le système catalytique est ensuite préparé par saturation de la solution par du chlorure d'hydrogène gazeux.

La réaction entre acétylène et chlorure d'hydrogène est réalisée de la manière suivante :

Un réacteur en pyrex de volume interne de 45 ml, muni d'une double enveloppe dans laquelle circule une huile caloporteuse et d'un dispositif d'introduction des réactifs constitué d'un embout en verre fritté destiné à assurer la dispersion des gaz dans le milieu liquide est chargé avec 30 ml d'une solution constituée de dodécylamine, de chlorure de palladium(II) et de solvant SHELLSOL K.

La solution est chauffée à 150° C et un flux gazeux renfermant un mélange de chlorure d'hydrogène et d'acétylène avec un rapport molaire HCl / $C_2H_2$ de 1,17 est introduit dans le réacteur. Le temps de séjour des gaz dans le réacteur, c'est-a-dire le rapport entre le volume du réacteur et le débit volumique des réactifs à la température de réaction est de 4,9 s. Le produit gazeux sortant du réacteur est analysé par chromatographie en phase gazeuse. Les seuls produits de réaction observés sont le chlorure de vinyle (VC) et le 1-chloroprène (1CPr). Les résultats sont rassemblés au tableau I. Le rendement est défini comme le rapport molaire entre le VC produit et l'acétylène introduit dans le réacteur. La sélectivité est calculée comme le rapport molaire entre le VC produit et la somme [ VC + (2 x 1CPr) ].

TABLEAU I

| N° ex. | Rapport pondéral dodécylamine / solvant SHELLSOL K | Rendement (%) | VC produit ($g.h^{-1}.l^{-1}$) | Sélectivité (%) | Remarques |
|---|---|---|---|---|---|
| 1 | 50 / 50 | 42,4 | 258 | 99,2 | |
| 2 | 25 / 75 | 59,4 | 361 | 97,5 | |
| 3 | 10 / 90 | 57,3 | 348 | 96,0 | légère dégradation du milieu réactionnel |

Exemples 4 à 5

Deux systèmes catalytiques sont préparés de la même manière que dans l'exemple 1 avec des quantités variables de dodécylamine et de solvant SHELLSOL K, mais le chlorure de palladium est remplacé par 15 mmol/l de chlorure de platine(II).

La réaction d'hydrochloration de l'acétylène est effectuée dans les mêmes conditions qu'aux exemples 1 à 3. Les résultats sont rassemblés au tableau II.

TABLEAU II

| N° ex. | Rapport pondéral dodécylamine / solvant SHELLSOL K | Rendement (%) | VC produit $(g.h^{-1}.l^{-1})$ | Sélectivité (%) |
|---|---|---|---|---|
| 4 | 25 / 75 | 5,1 | 31 | 98,9 |
| 5 | 10 / 90 | 7,6 | 46,2 | 97,0 |

Exemple 6(C)

Un système catalytique est préparé de la même manière que dans l'exemple 1, mais en absence de solvant organique dans la solution. Le système catalytique obtenu par saturation de la solution est solide, même à 150° C, ce qui rend impossible la réalisation de la réaction d'hydrochloration de l'acétylène dans le réacteur utilisé.

Exemple 7(C)

Un système catalytique est préparé de la même manière que dans l'exemple 1, mais en absence de dodécylamine dans la solution.

La réaction d'hydrochloration de l'acétylène est effectuée dans les mêmes conditions qu'aux exemples précédents. Les résultats sont rassemblés au tableau III.

Exemple 8(C)

Un système catalytique est préparé de la même manière que dans l'exemple 1, mais la dodécylamine est remplacée par de la diméthylformamide.

La réaction d'hydrochloration de l'acétylène est effectuée dans les mêmes conditions qu'aux exemples précédents. Les résultats sont rassemblés au tableau III.

TABLEAU III

| N° essai | Rendement (%) | VC produit $(g.h^{-1}.l^{-1})$ | Sélectivité (%) | Remarques |
|---|---|---|---|---|
| 7(C) | 0,3 | 2 | n.d. | Dégradation du milieu réactionnel |
| 8(C) | 19 | 116 | 93,2 | Dégradation du milieu réactionnel |

**Revendications**

1. Système catalytique d'hydrochloration comprenant au moins un composé de metal du groupe VIII, un chlorhydrate d'amine grasse comprenant plus de 8 atomes de carbone dont le point de fusion est supe'rieur à 25° C et un solvant organique choisi parmi les hydrocarbures aliphatiques, cycloaliphatiques et aromatiques et leurs mélanges.

2. Système catalytique selon la revendication 1 caractérisé en ce que le chlorhydrate d'amine renferme de 10 à 20 atomes de carbone.

3. Système catalytique selon une quelconque des revendications 1 ou 2 caractérisé en ce que le composé de métal du groupe VIII est choisi parmi les composés du palladium et les composés du platine.

4. Système catalytique selon une quelconque des revendications 1 à 3 caractérisé en ce que le rapport volumique entre le solvant et le chlorhydrate d'amine grasse varie d'environ 0,1 à environ 20 et en ce que la teneur en composé de métal du groupe VIII exprimée en millimoles par litre de système catalytique est supérieure ou égale à environ 1 mmol/l et inférieure ou égale à environ 200 mmol/l.

5. Procédé de fabrication de chlorure de vinyle par réaction de l'acétylène avec du chlorure d'hydrogène en présence d'un système catalytique liquide caractérisé en ce que le système catalytique comprend au moins un composé de métal du groupe VIII, un chlorhydrate d'amine grasse dont le point de fusion est supérieur à 25° C et un solvant organique choisi parmi les hydrocarbures aliphatiques, cycloaliphatiques et aromatiques et leurs mélanges.

6. Procédé selon la revendication 5 caractérisé en ce que le chlorhydrate d'amine grasse contient de 10 à 20 atomes de carbone.

7. Procédé selon une quelconque des revendications 5 ou 6 caractérisé en ce que le composé de métal du groupe VIII est choisi parmi les composés du palladium et les composés du platine.

8. Procédé selon une quelconque des revendications 5 à 7 caractérisé en ce que le rapport volumique entre le solvant et le chlorhydrate d'amine grasse varie d'environ 0,1 à environ 20 et en ce que la teneur en composé de métal du groupe VIII exprimée en millimoles par litre de système catalytique est supérieure ou égale à environ 1 mmol/l et inférieure ou égale à environ 200 mmol/l.

9. Procédé selon une quelconque des revendications 5 à 8 caractérisé en ce que la réaction est effectuée à une température d'environ 80° C à environ 180° C.

10. Procédé selon une quelconque des revendications 5 à 9 caractérisé en ce que le chlorure d'hydrogène et l'acétylène sont mis en oeuvre dans un rapport molaire d'environ 0,5 à environ 3.

## Claims

1. Catalytic hydrochlorination system comprising at least one group VIII metal compound and a fatty amine hydrochloride containing more than 8 carbon atoms, whose melting point is greater than 25°C and an organic solvent chosen from the aliphatic, cyclo-aliphatic and aromatic hydrocarbons and their mixtures.

2. Catalytic system according to Claim 1, characterised in that the amine hydrochloride contains from 10 to 20 carbon atoms.

3. Catalytic system according to any one of Claims 1 to 2, characterised in that the group VIII metal compound is chosen from palladium compounds and platinum compounds.

4. Catalytic system according to any one of Claims 1 to 3, characterised in that the volume ratio between the solvent and the fatty amine hydrochloride varies from approximately 0.1 to approximately 20 and in that the content of group VIII metal compound expressed in millimoles per litre of catalytic system is greater than or equal to approximately 1 mmol/l and less than or equal to approximately 200 mmol/l.

5. Process for the manufacture of vinyl chloride by reacting acetylene with hydrogen chloride in the presence of a liquid catalytic system, characterised in that the catalytic system comprises at least one group VIII metal compound, a fatty amine hydrochloride whose melting point is greater than 25°C and an organic solvent chosen from the aliphatic, cycloaliphatic and aromatic hydrocarbons and their mixtures.

6. Process according to Claim 5, characterised in that the fatty amine hydrochloride contains from 10 to 20 carbon atoms.

7. Process according to any one of Claims 5 and 6, characterised in that the group VIII metal compound is chosen from palladium compounds and platinum compounds.

8. Process according to any one of Claims 5 to 7, characterised in that the volume ratio between the solvent and the fatty amine hydrochloride varies from approximately 0.1 to approximately 20 and in that the content of group VIII metal compound expressed in millimoles per litre of catalytic system is greater than or equal to approximately 1 mmol/l and less than or equal to approximately 200 mmol/l.

9. Process according to any one of Claims 5 to 8, characterised in that the reaction is carried out at a temperature from approximately 80°C to approximately 180°C.

10. Process according to any one of Claims 5 to 9, characterised in that the hydrogen chloride and the acetylene are used in a molar ratio of approximately 0.5 to approximately 3.

**Patentansprüche**

1. Katalytisches System zur Hydrochlorierung, das wenigstens eine Verbindung eines Metalls der Gruppe VIII, ein Fettaminhydrochlorid mit mehr als 8 Kohlenstoffatomen, dessen Schmelzpunkt höher als 25 °C ist, und ein organisches Lösungsmittel, das unter den aliphatischen, cycloaliphatischen und aromatischen Kohlenwasserstoffen und ihren Gemischen ausgewählt ist, umfaßt.

2. Katalytisches System gemäß Anspruch 1, dadurch gekennzeichnet, daß das Aminhydrochlorid 10 bis 20 Kohlenstoffatome enthält.

3. Katalytisches System gemäß einem der Ansprüche 1 oder 2, dadurch gekennzeichnet, daß die Verbindung des Metalls der Gruppe VIII unter den Verbindungen des Palladiums und den Verbindungen des Platins ausgewählt ist.

4. Katalytisches System gemäß einem der Ansprüche 1 bis 3, dadurch gekennzeichnet, daß das Volumenverhältnis zwischen dem Lösungsmittel und dem Fettaminhydrochlorid von etwa 0,1 bis etwa 20 variiert und dadurch, daß der Gehalt an Verbindung des Metalls der Gruppe VIII, ausgedrückt in Millimol pro Liter katalytisches System, größer oder gleich etwa 1 mmol/l und kleiner oder gleich etwa 200 mmol/l ist.

5. Verfahren zur Herstellung von Vinylchlorid durch Reaktion von Acetylen mit Chlorwasserstoff in Gegenwart eines flüssigen katalytischen Systems, dadurch gekennzeichnet, daß das katalytische System wenigstens eine Verbindung eines Metalls der Gruppe VIII, ein Fettaminhydrochlorid, dessen Schmelzpunkt größer als 25 °C ist, und ein organisches Lösungsmittel, das unter den aliphatischen, cycloaliphatischen und aromatischen Kohlenwasserstoffen ausgewählt ist, umfaßt.

6. Verfahren gemäß Anspruch 5, dadurch gekennzeichnet, daß das Fettaminhydrochlorid 10 bis 20 Kohlenstoffatome enthält.

7. Verfahren gemäß einem der Ansprüche 5 oder 6, dadurch gekennzeichnet, daß die Verbindung des Metalls der Gruppe VIII unter den Verbindungen des Palladiums und den Verbindungen des Platins ausgewählt ist.

8. Verfahren gemäß einem der Ansprüche 5 bis 7, dadurch gekennzeichnet, daß das Volumenverhältnis zwischen dem Lösungsmittel und dem Fettaminhydrochlorid von etwa 0,1 bis etwa 20 variiert und dadurch, daß der Gehalt an Verbindung des Metalls der Gruppe VIII, ausgedrückt in Millimol pro Liter katalytisches System, größer oder gleich etwa 1 mmol/l und kleiner oder gleich etwa 200 mmol/l ist.

9. Verfahren gemaß einem der Ansprüche 5 bis 8, dadurch gekennzeichnet, daß die Reaktion bei einer Temperatur von etwa 80 °C bis etwa 180 °C ausgeführt wird.

10. Verfahren gemäß einem der Ansprüche 5 bis 9, dadurch gekennzeichnet, daß Chlorwasserstoff und Acetylen in einem Molverhältnis von etwa 0,5 bis etwa 3 verwendet werden.